# EUROPEAN PATENT SPECIFICATION

(11) **EP 3 322 426 B1**
(45) Date of publication and mention of the grant of the patent: **28.10.2026**
(21) Application number: 16736178.1
(22) Date of filing: 07.07.2016
(51) Int. Cl.: A61K 35/745, A23L 33/135, A61K 35/74, A61P 9/00

(54) **BIFIDOBACTERIA FOR TREATING CARDIAC CONDITIONS**
BIFIDOBAKTERIEN ZUR BEHANDLUNG VON HERZKRANKHEITEN
BIFIDOBACTÉRIES POUR LE TRAITEMENT DE PATHOLOGIES CARDIAQUES

(30) Priority: 16.07.2015 US 201562193537 P; 12.08.2015 GB 201514303
(43) Date of publication of application: 23.05.2018
(73) Proprietor: International N&H Denmark ApS, 2800 Kgs. Lyngby (DK)
(72) Inventor: STENMAN, Lotta, 02460 Kantvik (FI); LAHTINEN, Sampo, 08350 Lohja (FI); KONHILAS, John, Tucson, AZ 85719 (US)
(74) Representative: International N&H EMEA
(86) International application number: PCT/EP2016/066184
(87) International publication number: WO 2017/009187

(56) References cited:
- EP-A1- 2 808 024
- EP-B1- 2 808 024
- US-A1- 2004 126 356
- DATABASE WPI Week 201477, Derwent World Patents Index; AN 2014-U97769, XP002760964
- DATABASE WPI Week 200981, Derwent World Patents Index; AN 2009-R75690, XP002760965
- DATABASE WPI Week 200403, Derwent World Patents Index; AN 2004-027973, XP002760966
- EJTAHED H S ET AL: "Effect of probiotic yogurt containing Lactobacillus acidophilus and Bifidobacterium lactis on lipid profile in individuals with type 2 diabetes mellitus.", JOURNAL OF DAIRY SCIENCE JUL 2011, vol. 94, no. 7, July 2011 (2011-07-01), pages 3288 - 3294, XP055296947, ISSN: 1525-3198
- ALHAJ O A ET AL: "Hypocholesterolaemic effect of Bifidobacterium animalis subsp. lactis (Bb12) and trypsin casein hydrolysate", FOOD CHEMISTRY, ELSEVIER LTD, NL, vol. 123, no. 2, 15 November 2010 (2010-11-15), pages 430 - 435, XP027086085, ISSN: 0308-8146, [retrieved on 20100613]
- L.K. STENMAN ET AL: "Potential probiotic Bifidobacterium animalis ssp. lactis 420 prevents weight gain and glucose intolerance in diet-induced obese mice", BENEFICIAL MICROBES, vol. 5, no. 4, 1 December 2014 (2014-12-01), NL, pages 437 - 445, XP055296829, ISSN: 1876-2883, DOI: 10.3920/BM2014.0014
- SARVIN SANAIE ET AL: "Effect of a Probiotic Preparation (VSL#3) on Cardiovascular Risk Parameters in Critically-Ill Patients", JOURNAL OF CARDIOVASCULAR AND THORACIC RESEARCH, 1 April 2013 (2013-04-01), Tabriz, pages 67 - 70, XP055296823, Retrieved from the Internet <URL:http://www.ncbi.nlm.nih.gov/pmc/articles/PMC3825383/pdf/jcvtr-5-67.pdf> [retrieved on 20130410], DOI: 10.5681/jcvtr.2013.014

## Description

### Field of the Invention

This invention relates to new dosages of *Bifidobacteria animalis subsp. lactis strain 420* , and to use in food products, feed products, dietary supplements and pharmaceutical formulations containing them.

### Description of the Prior Art

Cardiovascular disease (CVD) remains the leading cause of death in industrialized countries (30% of all global deaths; ref. WHO Fact sheet number 317, Cardiovascular diseases) with 45% of these deaths due to coronary heart disease. Acute coronary events (ACEs) such as myocardial infarction (MI) and/or sudden cardiac death often result from atherosclerotic plaque rupture and the last 15-20 years of research has established a mechanistic link between inflammation in every aspect of the atherosclerotic process including plaque development, rupture and subsequent ACE (Shah PK. Inflammation and plaque vulnerability. Cardiovasc Drugs Ther 2009; 23: 31-40).

The use of microorganisms in treating cardiovascular conditions is in general known in the art.

WO 2010/145648 describes generally the use of *Bifidobacteria* to treat a number of cardiovascular conditions, including congestive heart failure and myocardial infarction. In particular, WO 2010/145648 specifically describes the administration of *Bifidobacteria* to mice for a period of 4 weeks, and the effects of the bacteria on glucose tolerance, plasma insulin, weight gain and tissue inflammation.

EP2808024 discloses *Bifidobacteria* for use in treating cardiovascular conditions such as aneurysm, angina, atherosclerosis etc. An example of a suitable *Bifidobacterium* disclosed is *B*. *animalis.* However, EP2808024 does not disclose any administration period for the *Bifidobacteria.*

Glück and Gebbers, Am. J. Clin. Nutr. 2003, 77, 517-520, describe the administration of a mixture of probiotics, including *Bifidobacteria,* to humans daily for a period of 3 weeks. The ingested probiotics reduced nasal colonization of pathogenic bacteria such as *Staphylococcus aureus* and *Streptococcus pneumoniae.*

It is generally considered by those skilled in the art that probiotics must be administered for a period of at least 3 weeks to enable them to colonise the gut and exert any therapeutic effect.

### Summary of the Invention

The present invention relates to a bacterium of the species Bifidobacterium animalis subsp. lactis strain 420 for use in reduzing the size of myocardial infarction in a mammal, wherein the bacterium is administered for a period of at least 7 days and not exceeding 18 days, and wherein the Bifidobacterium strain is to be administered at a dosage of 10⁶ to10¹² CFU of bacteria/dose ; wherein administration for 7-18 days reduces myocardial infarct size by at least 10% in comparison with a mammal that has been treated.

### Brief Description of the Drawings

Figure 1 illustrates the infarct size shown as representative histology in high-fat diet (HFD)-fed mice following a 10-day short term treatment with *Bifidobacterium animalis* subsp. *lactis* strain 420 (B420) according to the present invention (Example 1) and saline (control);
Figure 2 is a graph comparing infarct size (AON/AAR) in HFD-fed mice treated with B420 according to the present invention (Example 1) and saline (control); and
Figure 3 illustrates the infarct size in mice gavaged in a 5-day on-off protocol with B420 according to Comparative Example 1.

### Detailed Description of the Invention

### Bacteria

The bacterium used in the present invention is *Bifidobacterium animalis subsp. lactis* strain 420.

Preferably the *Bifidobacterium* to be used in the present invention is a *Bifidobacterium* which is generally recognised as safe and, which is preferably GRAS approved.

The bacterium may be used in any form capable of exerting the effects described herein.

The bacteria may comprise whole bacteria or may comprise bacterial components. Examples of such components include bacterial cell wall components such as peptidoglycan, bacterial nucleic acids such as DNA and RNA, bacterial membrane components, and bacterial structural components such as proteins, carbohydrates, lipids and combinations of these such as lipoproteins, glycolipids and glycoproteins.

Preferably the bacteria comprise whole viable bacteria.

*Bifidobacterium* for use disclosed herein is one which is suitable for human and/or animal consumption. A skilled person will be readily aware of specific species and or strains of *Bifidobacteria* from within the genera described herein which are used in the food and/or agricultural industries and which are generally considered suitable for human and/or animal consumption.

Described herein are *Bifidobacterium* used that may be of the same type (species and strain) or may comprise a mixture of species and/or strains.

Disclosed *Bifidobacteria* are from the species, *Bifidobacterium bifidium, Bifidobacterium longum, Bifidobacterium animalis, Bifidobacterium breve, Bifidobacterium infantis, Bifidobacterium catenulatum, Bifidobacterium pseudocatenulatum, Bifidobacterium adolescentis,* and *Bifidobacterium angulatum,* and combinations of any thereof.

The *Bifidobacterium* used in the present invention is of of the species *Bifidobacterium animalis* subsp. lactis strain 420 (B420). This strain is available from DuPont Nutrition Biosciences ApS. This strain of *Bifidobacterium animalis* subsp. *lactis* has also been deposited under the reference DGCC420 by DuPont Nutrition Biosciences ApS, of Langebrogade 1, 1411 Copenhagen K, Denmark, in accordance with the Budapest Treaty on 30 June 2015 at the Leibniz-Institut Deutsche Sammlung von Mikroorganismen und Zellkulturen GmbH (DSMZ), Inhoffenstrasse 7B, 38124 Braunschweig, Germany, where it is recorded under registration number DSM 32073.

In some embodiments, the *Bifidobacterium* is used in the present invention together with another probiotic microorganism. Examples of probiotic microorganisms include probiotic bacteria (as defined and exemplified above) and probiotic fungi. Preferably the probiotic microorganisms are probiotic bacteria.

In one embodiment, the additional probiotic bacterium is another bacterium of the genus *Bifidobacterium,* as defined and exemplified above. In another embodiment, the additional probiotic bacterium is a bacterium of a different genus and/or species. Examples of suitable probiotic bacteria include *Akkermansia ssp.* (such as *Akkermansia muciniphila), Acetobacter spp.* (such as *Acetobacter xylinum, Acetobacter hansenii, Acetobacter pasteurianus* and *Acetobacter aceti*), *Faecalibacterium ssp.* (such as *Faecalibacterium prausnitzii), Leuconostoc spp., Bacillus spp., Lactobacillus spp., Streptococcus thermophilus* or *Pediococcus sp.*

In one embodiment, the additional probiotic bacterium is a bacterium of the genus *Lactobacillus.*

Typically, the *Lactobacillus* bacteria are selected from the species *Lactobacillus acidophilus, Lactobacillus casei, Lactobacillus kefiri, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sakei, Lactobacillus reuteri, Lactobacillus fermentum, Lactobacillus farciminis, Lactobacillus lactis, Lactobacillus delbreuckii, Lactobacillus plantarum, Lactobacillus paraplantarum, Lactobacillus crispatus, Lactobacillus gasseri, Lactobacillus johnsonii* and *Lactobacillus jensenii,* and combinations of any thereof.

In preferred embodiments, the *Lactobacillus* bacterium used in the present invention is a probiotic *Lactobacillus.*

Preferably, the *Lactobacillus* bacterium used in the present invention of the species *Lactobacillus acidophilus.*

In one embodiment, the *Bifidobacterium* is used in the present invention together with a bacterium of the species *Lactobacillus acidophilus* strain NCFM. *Lactobacillus acidophilus* NCFM was deposited by Rhodia Chimie, France, at the American Type Culture Collection as PTA-4797 on 15 November 2002.

### Dosage

The *Bifidobacterium animalis* subsp. *lactis* strain 420 (B420) is dosed in accordance with the present invention for a period of at least 7 days and not exceeding 18 days.

It has surprisingly been found by the present inventors that, when dosed for the above period, the *Bifidobacterium* exerts unexpectedly favourable cardiac effects. In particular, it has been shown that mammals treated with *Bifidobacterium* for the above period exhibit improvement in various parameters relating to cardiac function compared with mammals which have not been treated with *Bifidobacterium.* This confers the potential for administration of *Bifidobacterium* to prevent, or reduce the probability of suffering from, and/or reduce the impact of, and/or the consequences of myocardial infarction.

The improved effect of treatment with *Bifidobacterium* for the above dosing period runs contrary to the teaching of the prior art. It is generally considered by those skilled in the art that probiotics need to be dosed for at least 3 weeks before they can colonise the gut and exhibit any therapeutic effect. It would not therefore have been expected that the *Bifidobacterium* could exhibit any therapeutic effect when dosed for a much shorter period.

The *Bifidobacterium animalis* subsp. *lactis* strain 420 (B420) is dosed in accordance with the present invention for a minimum period of at least 7 days, such as at least 8 days, such as at least 9 days, such as at least 10 days, such as at least 11 days, such as at least 12 days, such as at least 13 days, such as at least 14 days.

The *Bifidobacterium animalis* subsp. *lactis* strain 420 (B420) is dosed in accordance with the present invention for a maximum period of 18 days, such as 17 days, such as 16 days, such as 15 days, such as 14 days, such as 13 days, such as 12 days, such as 11 days.

In one embodiment, the *Bifidobacterium animalis* subsp. *lactis* strain 420 (B420), is dosed in accordance with the present invention for a period of at least 9 days and not exceeding 14 days.

In one embodiment, the *Bifidobacterium animalis* subsp. *lactis* strain 420 (B420) is dosed in accordance with the present invention for a period of at least 10 days and not exceeding 12 days.

The frequency of dosing of the *Bifidobacterium animalis* subsp. *lactis* strain 420 (B420) varies depending on the subject to be treated. Typically, the *Bifidobacterium animalis* subsp. *lactis* strain 420 (B420) is dosed in accordance with the present invention once, twice, three or four times per day for the specified period. Preferably, the *Bifidobacterium animalis* subsp. *lactis* strain 420 (B420) is dosed in accordance with the present invention once per day for the specified period.

The *Bifidobacterium animalis* subsp. *lactis* strain 420 (B420), bacteria used in accordance with the present invention is administered at a dosage of from about 10⁶ to about 10¹² CFU of bacteria /dose, preferably about 10⁷ to about 10¹¹ CFU of bacteria /dose. By the term "per dose" it is meant that this amount of bacteria is provided to a subject either per day or per intake, preferably per day. For example, if the bacteria are to be administered in a food product (for example in a yoghurt) - then the food product, e.g. yoghurt will preferably contain from about 10⁶ to 10¹² CFU, more preferably about 10⁷ to about 10¹¹ CFU, and most preferably about 10⁹ to about 10¹⁰ CFU of the bacteria. If the bacteria are to be administered in a dietary supplement, then the dietary supplement will preferably contain about 10⁸ to about 10¹² CFU, more preferably about 10⁹ to 10¹¹ CFU of the bacteria. If the bacteria are to be administered in a pharmaceutical composition, then the pharmaceutical composition will preferably contain about 10⁸ to about 10¹² CFU, more preferably about 10⁹ to 10¹¹ CFU of the bacteria. Alternatively, however, this amount of bacteria may be split into multiple administrations each consisting of a smaller amount of microbial loading - so long as the overall amount of bacteria received by the subject in any specific time (for instance each 24 hour period) is within the ranges specified above.

The concentration of the bacteria per g of support varies depending on the intended dose and the nature of the support. In one embodiment, the *Bifidobacterium animalis* subsp. *lactis* strain 420 (B420), bacteria used in accordance with the present invention may be present in a concentration of from 5000 to 10¹³ CFU of bacteria/ g of support, preferably about 5 x 10⁶ to about 10¹² CFU of bacteria / g of support. When the support is a food product, such as a yogurt, the bacteria are preferably present in a concentration of from about 5000 to about 5 x 10⁸ CFU of bacterial g of food product, preferably about 5 x 10⁶ to about 5 x 10⁷ CFU of bacteria / g of food product. When the support is a dietary supplement, the bacteria are preferably present in a concentration of from 10⁹ to about 10¹³ CFU / g of dietary supplement, more preferably about 10¹⁰ to 10¹² CFU / g of dietary supplement. When the support is a pharmaceutical composition, the bacteria are preferably present in a concentration of from 10⁹ to about 10¹³ CFU / g of dietary supplement, more preferably about 10¹⁰ to 10¹² CFU / g of pharmaceutical composition.

In one embodiment, preferably the *Bifidobacterium animalis* subsp. *lactis* strain 420 (B420), may be administered once per day at a dosage of from about 10⁶ to about 10¹² CFU of bacteria /day, preferably about 10⁸ to about 10¹² CFU of bacteria /day. Hence, the effective amount in this embodiment may be from about 10⁶ to about 10¹² CFU of bacteria /day, preferably about 10⁷ to about 10¹¹ CFU of bacteria / day For example, if the bacteria are to be administered in a food product (for example in a yoghurt) - then the bacteria will preferably be administered once per day in a dosage of about 10⁶ to 10¹² CFU of bacteria / day, more preferably about 10⁷ to about 10¹¹ CFU of bacteria / day and most preferably about 10⁹ to about 10¹⁰ CFU of the bacteria / day. If the bacteria are to be administered in a dietary supplement, then the bacteria will preferably be administered once per day in a dosage of about 10⁸ to about 10¹² CFU of bacteria / day, more preferably about 10⁹ to 10¹¹ CFU of the bacteria / day. If the bacteria are to be administered in a pharmaceutical composition, then the bacteria will preferably be administered once per day in a dosage of about 10⁸ to about 10¹² CFU bacteria / day, more preferably about 10⁹ to 10¹¹ CFU of the bacteria / day.

CFU stands for "colony-forming units". By 'support' is meant the food product, dietary supplement or the pharmaceutically acceptable support.

When one or more other microorganisms (such as bacteria, preferably probiotic bacteria) are used in combination with the *Bifidobacterium animalis* subsp. *lactis* strain 420 (B420) bacteria in accordance with the present invention, the other bacteria may similarly be administered at a dosage of from about 10⁶ to about 10¹² CFU of microorganism/dose, preferably about 10⁷ to about 10¹¹ CFU of microorganism/dose. Depending on the manner in which the microorganisms are administered (food product, dietary supplement, or pharmaceutical composition), the preferred amounts of the one or more other microorganisms will typically be in the ranges specified above for *Bifidobacteria.*

### Subjects / Medical Indications

The *Bifidobacteria* (and any additional probiotic microorganisms, if present) to which the present invention relates are administered to a mammal, including for example livestock (including cattle, horses, pigs, chickens and sheep), and humans. In some aspects of the present invention the mammal is a companion animal (including pets), such as a dog or a cat for instance. In some aspects of the present invention, the subject may suitably be a human.

The *Bifidobacteria* (and any additional probiotic microorganisms, if present) disclosed herein may be suitable for treating a number of diseases or conditions in mammals (particularly humans). In this specification the term "treatment" or "treating" refers to any administration of the *Bifidobacteria* (and any additional probiotic microorganisms, if present) according to the present invention and includes: (1) preventing the specified disease from occurring in a mammal which may be predisposed to the disease but does not yet experience or display the pathology or symptomatology of the disease (including prevention of one or more risk factors associated with the disease); (2) inhibiting the disease in a mammal that is experiencing or displaying the pathology or symptomatology of the diseased (*i.e.,* arresting further development of the pathology and/or symptomatology), or (3) ameliorating the disease in a mammal that is experiencing or displaying the pathology or symptomatology of the diseased (*i.e.,* reversing the pathology and/or symptomatology).

In particular, the use of *Bifidobacteria* according to the present invention is suitable for the treatment of mammals ingesting a high-fat diet. This aspect is discussed in more detail below.

Examples of cardiovascular diseases disclosed herein to be treatable by use of the *Bifidobacteria* (and any additional probiotic microorganisms, if present) include aneurysm, angina, atherosclerosis, cerebrovascular accident (stroke), cerebrovascular disease, congestive heart failure (CHF), dilated cardiomyopathy, inflammatory heart disease (including endocarditis, inflammatory cardiomegaly and myocarditis), coronary artery disease, myocardial infarction (heart attack) and peripheral vascular disease.

An aneurysm is a localized, blood-filled dilation (balloon-like bulge) of a blood vessel caused by disease or weakening of the vessel wall. Aneurysms most commonly occur in arteries at the base of the brain (the circle of Willis) and in the aorta (the main artery coming out of the heart, a so-called aortic aneurysm). As the size of an aneurysm increases, there is an increased risk of rupture, which can result in severe hemorrhage or other complications including sudden death.

Angina pectoris, commonly known as angina, is severe chest pain due to ischemia (a lack of blood and hence oxygen supply) of the heart muscle, generally due to obstruction or spasm of the coronary arteries (the heart's blood vessels). Coronary artery disease, the main cause of angina, is due to atherosclerosis of the cardiac arteries.

Atherosclerosis is the condition in which an artery wall thickens as the result of a build up of fatty materials such as cholesterol. It is a syndrome affecting arterial blood vessels. It is a chronic inflammatory response in the walls of arteries, in large part due to the accumulation of macrophage white blood cells and promoted by low density (especially small particle) lipoproteins (plasma proteins that carry cholesterol and triglycerides) without adequate removal of fats and cholesterol from the macrophages by functional high density lipoproteins (HDL). It is commonly referred to as a hardening or furring of the arteries. It is caused by the formation of multiple plaques within the arteries.

A stroke is the rapidly developing loss of brain function(s) due to disturbance in the blood supply to the brain. This can be due to ischemia (lack of blood supply) caused by thrombosis or embolism or due to a hemorrhage. As a result, the affected area of the brain is unable to function, leading to inability to move one or more limbs on one side of the body, inability to understand or formulate speech, or see one side of the visual field and ultimately to death.

Cerebrovascular disease is a group of brain dysfunctions related to disease of blood vessels supplying the brain. Hypertension is the most important cause that damages the blood vessel lining endothelium exposing the underlying collagen where platelets aggregate to initiate a repairing process which is not always complete and perfect.

Sustained hypertension permanently changes the architecture of the blood vessels making them narrow, stiff, deformed and uneven which are more vulnerable to fluctuations of blood pressure. A fall in blood pressure during sleep can lead to marked reduction in blood flow in the narrowed blood vessels causing ischemic stroke in the morning whereas a sudden rise in blood pressure can cause tearing of the blood vessels causing intracranial hemorrhage during excitation at daytime. Primarily people who are elderly, diabetic, smoker, or have ischemic heart disease, have cerebrovascular disease. All diseases related to artery dysfunction can be classified under a disease as known as macrovascular disease. This is a simplistic study by which arteries are blocked by fatty deposits or by a blood clot. The results of cerebrovascular disease can include a stroke, or even sometimes a hemorrhagic stroke. Ischemia or other blood vessel dysfunctions can affect one during a cerebrovascular accident.

Heart failure is a global term for the physiological state in which cardiac output is insufficient for the body's needs. This may occur when the cardiac output is low (often termed "congestive heart failure"). Common causes of heart failure include myocardial infarction and other forms of ischemic heart disease, hypertension, valvular heart disease and cardiomyopathy.

Dilated cardiomyopathy is a condition in which the heart becomes enlarged and cannot pump blood efficiently. Dilated cardiomyopathy is the most common form of non-ischemic cardiomyopathy. About one in three cases of congestive heart failure (CHF) is due to dilated cardiomyopathy.

Coronary disease (or coronary heart disease) refers to the failure of coronary circulation to supply adequate circulation to cardiac muscle and surrounding tissue. It is most commonly equated with atherosclerotic coronary artery disease, but coronary disease can be due to other causes, such as coronary vasospasm. It is possible for the stenosis to be caused by the spasm.

Myocardial infarction, commonly known as a heart attack, occurs when the blood supply to part of the heart is interrupted causing some heart cells to die. This is most commonly due to occlusion (blockage) of a coronary artery following the rupture of a vulnerable atherosclerotic plaque, which is an unstable collection of lipids (like cholesterol) and white blood cells (especially macrophages) in the wall of an artery. The resulting ischemia (restriction in blood supply) and oxygen shortage, if left untreated for a sufficient period of time, can cause damage and/or death (infarction) of heart muscle tissue (myocardium).

*Bifidobacteria* (and any additional probiotic microorganisms, if present) are disclosed herein to be suitable for treating myocardial infarction in a mammal. In one embodiment the term "treating myocardial infarction" comprises preventing, or reducing the probability of suffering from, myocardial infarction. In one embodiment the term "treating myocardial infarction" comprises reducing the impact of, or the consequences of, myocardial infarction. In one embodiment the term "treating myocardial infarction" comprises reducing the size of the myocardial infarction. In one embodiment the term "treating myocardial infarction" comprises increasing or maintaining the ejection fraction subsequent to a myocardial infarction. In one embodiment the term "treating myocardial infarction" comprises decreasing or maintaining the left ventricular volume during systole subsequent to a myocardial infarction. In one embodiment the term "treating myocardial infarction" comprises increasing or maintaining the left ventricular posterior wall dimension during systole subsequent to a myocardial infarction. In each of the above embodiments the reduction, decrease or increase is at least 10%, at least 20%, at least 30%, at least 40%, at least 50%, at least 60%, at least 70%, at least 80%, at least 90% in comparison with a mammal that has not been treated with *Bifidobacteria* (and any additional probiotic microorganisms, if present) according to the present invention.

Peripheral vascular disease (PVD), also known as peripheral artery disease (PAD) or peripheral artery occlusive disease (PAOD), includes all diseases caused by the obstruction of large arteries in the arms and legs. PVD can result from atherosclerosis, inflammatory processes leading to stenosis, an embolism or thrombus formation. It causes either acute or chronic ischemia (lack of blood supply), typically of the legs.

### Diet

As noted above, diabetic and/or obese mammals treated with bacteria according to the present invention may ingest a high-fat diet while mitigating the metabolic consequences of their condition(s). In this specification the term 'high-fat diet' means a diet generally containing at least 20%, preferably at least 25%, such as at least 30%, for example at least 35%, such as at least 40%, for example at least 45%, such as at least 50%, for example at least 55%, such as at least 60%, for example at least 65%, such as at least 70%, for example at least 75%, such as at least 80%, for example at least 85%, such as at least 90% of calories from fat.

### Compositions

While it is possible to administer *Bifidobacteria* (and any additional probiotic microorganisms, if present) alone according to the present invention (i.e. without any support, diluent or excipient), the *Bifidobacteria* (and any additional probiotic microorganisms, if present) are typically and preferably administered on or in a support as part of a product, in particular as a component of a food product, a dietary supplement or a pharmaceutical formulation. These products typically contain additional components well known to those skilled in the art.

Any product which can benefit from the composition may be used in the present invention. These include but are not limited to foods, particularly fruit conserves and dairy foods and dairy food-derived products, and pharmaceutical products. The *Bifidobacteria* (and any additional probiotic microorganisms, if present) may be referred to herein as "the composition of the present invention" or "the composition".

### Food

In one embodiment, the *Bifidobacteria* (and any additional probiotic microorganisms, if present) are employed according to the invention in a food product such as a food supplement, a drink or a powder based on milk. Here, the term "food" is used in a broad sense - and covers food for humans as well as food for animals (i.e. a feed). In a preferred aspect, the food is for human consumption.

The food may be in the form of a solution or as a solid - depending on the use and/or the mode of application and/or the mode of administration.

When used as, or in the preparation of, a food, such as functional food, the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

By way of example, the composition of the present invention can be used as an ingredient to soft drinks, a fruit juice, energy drinks, sports drinks and sports nutritional supplements or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods (including snack bars), balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk, calcium fortified and non-fortified coffee beverage.

The composition can further be used as an ingredient in food products such as American cheese sauce, anti-caking agent for grated & shredded cheese, chip dip, cream cheese, dry blended whip topping fat free sour cream, freeze/thaw dairy whipping cream, freeze/thaw stable whipped tipping, low fat and light natural cheddar cheese, low fat Swiss style yoghurt, aerated frozen desserts, hard pack ice cream, label friendly, improved economics & indulgence of hard pack ice cream, low fat ice cream: soft serve, barbecue sauce, cheese dip sauce, cottage cheese dressing, dry mix Alfredo sauce, mix cheese sauce, dry mix tomato sauce and others.

The term "dairy product" as used herein is meant to include a medium comprising milk of animal and/or vegetable origin. As milk of animal origin there can be mentioned cow's, sheep's, goat's or buffalo's milk. As milk of vegetable origin there can be mentioned any fermentable substance of vegetable origin which can be used according to the invention, in particular originating from soybeans, rice or cereals.

Still more preferably the food product employed according to the invention is a fermented milk or humanized milk.

For certain aspects, preferably the present invention may be used in connection with yoghurt production, such as fermented yoghurt drink, yoghurt, drinking yoghurt, cheese, fermented cream, milk based desserts and others.

Suitably, the composition can be further used as an ingredient in one or more of cheese applications, meat applications, or applications comprising protective cultures.

The present invention also provides a method of preparing a food or a food ingredient, the method comprising admixing the composition according to the present invention with another food ingredient.

Advantageously, the present invention relates to products that have been contacted with the composition of the present invention (and optionally with other components/ingredients), wherein the composition is used in an amount to be capable of improving the nutrition and/or health benefits of the product.

As used herein the term "contacted" refers to the indirect or direct application of the composition of the present invention to the product. Examples of the application methods which may be used, include, but are not limited to, treating the product in a material comprising the composition, direct application by mixing the composition with the product, spraying the composition onto the product surface or dipping the product into a preparation of the composition.

Where the product of the invention is a foodstuff, the composition of the present invention is preferably admixed with the product. Alternatively, the composition may be included in the emulsion or raw ingredients of a foodstuff. In a further alternative, the composition may be applied as a seasoning, glaze, colorant mixture, and the like.

For some applications, it is important that the composition is made available on or to the surface of a product to be affected/treated. This allows the composition to impart one or more of the following favourable characteristics: nutrition and/or health benefits.

The compositions of the present invention may be applied to intersperse, coat and/or impregnate a product with a controlled amount of a microorganism.

Preferably, the composition is used to ferment milk or sucrose fortified milk or lactic media with sucrose and/or maltose where the resulting media containing all components of the composition - i.e. said microorganism according to the present invention - can be added as an ingredient to yoghurt milk in suitable concentrations - such as for example in concentrations in the final product which offer a daily dose of 10⁶-10¹¹ cfu. The microorganism according to the present invention may be used before or after fermentation of the yoghurt.

For some aspects the microorganisms according to the present invention are used as, or in the preparation of, animal feeds, such as livestock feeds, in particular poultry (such as chicken) feed, or pet food.

Advantageously, where the product is a food product, the *Bifidobacteria* (and any additional probiotic microorganisms, if present) should remain effective through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.

### Food Ingredient

The composition of the present invention may be used as a food ingredient and/or feed ingredient.

As used herein the term "food ingredient" or "feed ingredient" includes a formulation which is or can be added to functional foods or foodstuffs as a nutritional supplement.

The food ingredient may be in the form of a solution or as a solid - depending on the use and/or the mode of application and/or the mode of administration.

### Food Supplements

The composition of the present invention may be - or may be added to - food supplements (also referred to herein as dietary supplements).

### Functional Foods

The composition of the present invention may be - or may be added to - functional foods.

As used herein, the term "functional food" means food which is capable of providing not only a nutritional effect, but is also capable of delivering a further beneficial effect to consumer.

Accordingly, functional foods are ordinary foods that have components or ingredients (such as those described herein) incorporated into them that impart to the food a specific functional - e.g. medical or physiological benefit - other than a purely nutritional effect.

Although there is no legal definition of a functional food, most of the parties with an interest in this area agree that they are foods marketed as having specific health effects beyond basic nutritional effects.

Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine.

### Medicament

The term "medicament" as used herein encompasses medicaments for both human and animal usage in human and veterinary medicine. In addition, the term "medicament" as used herein means any substance which provides a therapeutic and/or beneficial effect. The term "medicament" as used herein is not necessarily limited to substances which need Marketing Approval, but may include substances which can be used in cosmetics, nutraceuticals, food (including feeds and beverages for example), probiotic cultures, and natural remedies. In addition, the term "medicament" as used herein encompasses a product designed for incorporation in animal feed, for example livestock feed and/or pet food.

### Pharmaceutical

The composition of the present invention may be used as - or in the preparation of - a pharmaceutical. Here, the term "pharmaceutical" is used in a broad sense - and covers pharmaceuticals for humans as well as pharmaceuticals for animals (i.e. veterinary applications). In a preferred aspect, the pharmaceutical is for human use and/or for animal husbandry.

The pharmaceutical can be for therapeutic purposes - which may be curative or palliative or preventative in nature. The pharmaceutical may even be for diagnostic purposes.

A pharmaceutically acceptable support may be for example a support in the form of compressed tablets, tablets, capsules, ointments, suppositories or drinkable solutions. Other suitable forms are provided below.

When used as - or in the preparation of - a pharmaceutical, the composition of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.

The pharmaceutical may be in the form of a solution or as a solid - depending on the use and/or the mode of application and/or the mode of administration.

The *Bifidobacteria* (and any additional probiotic microorganisms if present) of the present invention may be used as pharmaceutical ingredients. Here, the composition may be the sole active component or it may be at least one of a number (i.e. 2 or more) of active components.

The pharmaceutical ingredient may be in the form of a solution or as a solid - depending on the use and/or the mode of application and/or the mode of administration.

The *Bifidobacteria* (and any additional probiotic microorganisms, if present) may be used according to the present invention in any suitable form - whether when alone or when present in a combination with other components or ingredients. The lactic acid bacteria used in the present invention may be referred to herein as "the composition". Likewise, combinations comprising the composition of the present invention and other components and/or ingredients (i.e. ingredients - such as food ingredients, functional food ingredients or pharmaceutical ingredients) may be used in any suitable form.

The *Bifidobacteria* (and any additional probiotic microorganisms, if present) may be used according to the present invention in the form of solid or liquid preparations or alternatives thereof. Examples of solid preparations include, but are not limited to tablets, capsules, dusts, granules and powders which may be wettable, spray-dried or freeze-dried. Examples of liquid preparations include, but are not limited to, aqueous, organic or aqueous-organic solutions, suspensions and emulsions.

Suitable examples of forms include one or more of: tablets, pills, capsules, ovules, solutions or suspensions, which may contain flavouring or colouring agents, for immediate-, delayed-, modified-, sustained-, pulsed- or controlled-release applications.

By way of example, if the composition of the present invention is used in a tablet form - such for use as a functional ingredient - the tablets may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethylcellulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

The forms may also include gelatin capsules; fibre capsules, fibre tablets etc.; or even fibre beverages.

Further examples of form include creams. For some aspects the microorganism used in the present invention may be used in pharmaceutical and/or cosmetic creams such as sun creams and/or after-sun creams for example.

In one aspect, the composition according to the present invention may be administered in an aerosol, for example by way of a nasal spray, for instance for administration to the respiratory tract.

### Combinations

The composition of the present invention may additionally contain one or more prebiotics. Prebiotics are a category of functional food, defined as non-digestible food ingredients that beneficially affect the host by selectively stimulating the growth and/or activity of one or a limited number of bacteria (particularly, although not exclusively, probiotics, such as *Bifidobacteria* and/or *Lactobacillus*) in the colon, and thus improve host health. Typically, prebiotics are carbohydrates (such as oligosaccharides), but the definition does not preclude non-carbohydrates. The most prevalent forms of prebiotics are nutritionally classed as soluble fibre. To some extent, many forms of dietary fibre exhibit some level of prebiotic effect.

In one embodiment, a prebiotic is a selectively fermented ingredient that allows specific changes, both in the composition and/or activity in the gastrointestinal microflora that confers benefits upon host well-being and health.

Suitably, the prebiotic may be used according to the present invention in an amount of 0.01 to 100 g / day, preferably 0.1 to 50 g / day, more preferably 0.5 to 20 g / day. In one embodiment, the prebiotic may be used according to the present invention in an amount of 1 to 10 g / day, preferably 2 to 9 g / day, more preferably 3 to 8 g / day. In another embodiment, the prebiotic may be used according to the present invention in an amount of 5 to 50 g / day, preferably 10 to 25 g / day.

Examples of dietary sources of prebiotics include soybeans, inulin sources (such as Jerusalem artichoke, jicama, and chicory root), raw oats, unrefined wheat, unrefined barley and yacon.

Examples of suitable prebiotics include alginate, xanthan, pectin, locust bean gum (LBG), inulin, guar gum, galacto-oligosaccharide (GOS), fructo-oligosaccharide (FOS), polydextrose (i.e. Litesse^{®}), lactitol, lactosucrose, soybean oligosaccharides, isomaltulose (Palatinose^{™}), isomalto-oligosaccharides, gluco-oligosaccharides, xylo-oligosaccharides, manno-oligosaccharides, beta-glucans, cellobiose, raffinose, gentiobiose, melibiose, xylobiose, cyclodextrins, isomaltose, trehalose, stachyose, panose, pullulan, verbascose, galactomannans, and all forms of resistant starches. A particularly preferred example of a prebiotic is polydextrose.

In some embodiments, a combination of *Bifidobacterium* (and any additional probiotic microorganisms, if present) bacteria and prebiotics according to the present invention exhibits a synergistic effect in certain applications (i.e. an effect which is greater than the additive effect of the bacteria when used separately). Without wishing to be bound by theory, it is believed that such a combination is capable of selectively stimulating the growth and/or activity of the *Bifidobacteria* (and any additional probiotic microorganisms, if present) bacteria in the colon, and thus improve host health.

Suitably, the prebiotic used in the combination is polydextrose.

### Example 1

### Materials and methods

C57BI/6J mice were obtained from Jackson Laboratories.

A 10-day protocol was conducted to see whether the positive effect of B420 on infarct size can be seen before the probiotic colonizes the gut. At 10 weeks of age, mice were first fed a high-fat diet (58% of calories from fat) for two weeks before initiating a 10-day treatment, once per day, with vehicle or *Bifidobacterium animalis* ssp. *lactis 420* B420 (10⁹ CFU/day).

### Cardiac ischemia-reperfusion protocol

Following each gavage sequence, mice were subjected to the cardiac ischemia-reperfusion protocol. Mice were anesthetized with an intraperitoneal injection of 250 mg/kg tribromoethanol, intubated and ventilated with 0.5-2.0% isoflurane. To maintain body temperature and restore potential loss of fluid, 500 µl of warmed sterile saline was injected into the dorsal subcutaneous space. The heart was exposed and the left coronary artery was visualized following a left anterior thoracotomy. The left coronary artery was occluded using 7-0 suture compressing a small piece of tubing (PE-10) to prevent vessel damage during occlusion. After 30 minutes of occlusion, the ligature was removed and the animal was allowed to recover.

During the occlusion of the left coronary artery, the heart suffers ischemia, which is then reperfused after 30 minutes. This reperfusion causes inflammation in the ischemic regions of the heart.

### Histological assessment of infarct size

Following 3 days of reperfusion, mice were sacrificed and hearts were immediately sectioned (1 mm) and double-stained with Evans blue and triphenyltetrazolium chloride (TTC) to define the necrotic area and the area subjected to ischemia (area at risk). After incubation, infarcted myocardium stained white (Area of Necrosis), viable tissue within Area at Risk stained red, and perfused tissue remained blue. Infarct size was determined as Area of Necrosis vs. Area at Risk. The area of infarction had been validated by staining sections for IL-6 and ICAM-1 - the inflammatory markers correlated well with the infarcted area as determined with Evans blue staining.

### Results

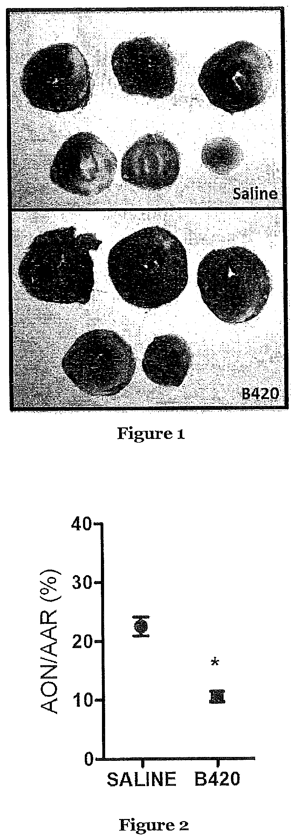

Figure 1 illustrates the infarct size in high-fat diet-fed mice following a 10-day short term treatment with B420 shown as representative histology. Figure 2 is a graph of infarct size with all data points included (N = 18 for saline, N = 13 for B420).

Although B420 would have likely not colonized the gut within 10 days of treatment, the results show B420 reduced infarct size in mice by more than 50%. This demonstrates that the probiotic can be very effective even in short-term treatment.

### Comparative Example 1

As a comparison, an on-off gavage protocol was conducted to see whether B420 ingestion needs to be continuous in order to reduce infarct size. Mice were first fed a high-fat diet for two weeks before the following sequence: 5 days gavage (10⁹ CFU/day), 5 days off, and the sequence was repeated 3 times. Mice were then gavaged for a final 5 days with B420.

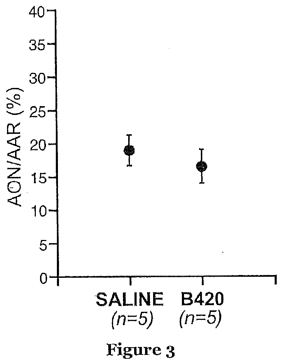

The results are shown in Figure 3 showing infarct size in mice gavaged on-off with B420. The results show that short 5-day gavages were insufficient to produce a similar reduction in infarct size in high-fat fed mice (Figure 3).

## Claims

1. A bacterium of the species *Bifidobacterium animalis subsp. lactis* strain 420 for use in reducing the size of the myocardial infarction in a mammal, wherein the bacterium is administered for a period of at least 7 days and not exceeding 18 days, and wherein the *Bifidobacteria* is to be administered at a dosage of 10⁶ to 10¹² CFU of bacteria/dose; wherein administration for 7-18 days reduces myocardial infarct size by at least 10% in comparison with a mammal that has not been treated.

2. The bacterium for use of claim 1, wherein the bacterium is to be administered for a period of at least 9 days and not exceeding 14 days.

3. The bacterium for use of any preceding claim, wherein the bacterium is to be administered once a day for the administration period.

4. The bacterium for use of any preceding claim, wherein the mammal in need of the treatment ingests a high-fat diet.

5. The bacterium for use of any preceding claim, wherein the *Bifidobacteria* is to be administered as a component of a food product, a dietary supplement or a pharmaceutical composition.

6. The bacterium for use of claim 5, wherein the *Bifidobacteria* is to be administered as a component of a yogurt.

7. The bacterium for use of claim 5, wherein the *Bifidobacteria* is to be administered as a component of a dietary supplement.

8. The bacterium for use of claim 5, wherein the *Bifidobacteria* is to be administered as a component of a pharmaceutical composition.

9. The bacterium for use of any preceding claim, wherein the *Bifidobacteria* is to be administered at a dosage of 10⁹ to 10¹¹ CFU of bacteria/dose.

10. The bacterium for use of claim 6, wherein the yoghurt contains from 10⁸ to 10¹² CFU of the bacteria per dose.

## Patentansprüche

1. Bakterium der Spezies *Bifidobacterium animalis subsp. lactis*, Stamm 420, zur Verwendung bei der Verringerung der Größe des Myokardinfarkts bei einem Säuger, wobei das Bakterium über einen Zeitraum von mindestens 7 und höchstens 18 Tagen verabreicht wird und wobei die Bifidobakterien in einer Dosierung von 10⁶ bis 10¹² CFU Bakterien/Dosis zu verabreichen sind; wobei durch eine 7- bis 18-tägige Verabreichung die Myokardinfarktgröße um mindestens 10 % im Vergleich mit einem Säuger, der nicht behandelt wurde, reduziert wird.

2. Bakterium zur Verwendung nach Anspruch 1, wobei das Bakterium über einen Zeitraum von mindestens 9 und höchstens 14 Tagen zu verabreichen ist.

3. Bakterium zur Verwendung nach einem vorhergehenden Anspruch, wobei das Bakterium einmal täglich über den Verabreichungszeitraum zu verabreichen ist.

4. Bakterium zur Verwendung nach einem vorhergehenden Anspruch, wobei sich der die Behandlung benötigende Säuger fettreich ernährt.

5. Bakterium zur Verwendung nach einem vorhergehenden Anspruch, wobei die Bifidobakterien als Komponente eines Lebensmittelprodukts, eines Nahrungsergänzungsmittels oder einer pharmazeutischen Zusammensetzung zu verabreichen sind.

6. Bakterium zur Verwendung nach Anspruch 5, wobei die Bifidobakterien als Komponente eines Joghurts zu verabreichen sind.

7. Bakterium zur Verwendung nach Anspruch 5, wobei die Bifidobakterien als Komponente eines Nahrungsergänzungsmittels zu verabreichen sind.

8. Bakterium zur Verwendung nach Anspruch 5, wobei die Bifidobakterien als Komponente einer pharmazeutischen Zusammensetzung zu verabreichen sind.

9. Bakterium zur Verwendung nach einem vorhergehenden Anspruch, wobei die Bifidobakterien in einer Dosierung von 10⁹ bis 10¹¹ CFU Bakterien/Dosis zu verabreichen sind.

10. Bakterium zur Verwendung nach Anspruch 6, wobei der Joghurt 10⁸ bis 10¹² CFU der Bakterien pro Dosis enthält.

## Revendications

1. Bactérie de la souche 420 de l'espèce Bifidobacterium animalis subsp. lactis destinée à être utilisée pour réduire la taille de l'infarctus du myocarde chez un mammifère, dans laquelle la bactérie est administrée pendant une période d'au moins 7 jours et ne dépassant pas 18 jours, et dans laquelle les bifidobactéries doivent être administrées à une dose de 10⁶ à 10¹² UFC de bactéries/dose ; dans laquelle l'administration pendant 7-18 jours réduit la taille de l'infarctus du myocarde d'au moins 10 % par rapport à un mammifère qui n'a été traité.

2. Bactérie pour utilisation selon la revendication 1, dans laquelle la bactérie doit être administrée pendant une période d'au moins 9 jours et ne dépassant pas 14 jours.

3. Bactérie pour utilisation selon l'une quelconque des revendications précédentes, dans laquelle la bactérie doit être administrée une fois par jour pendant la période d'administration.

4. Bactérie pour utilisation selon l'une quelconque des revendications précédentes, dans laquelle le mammifère ayant besoin du traitement ingère un régime alimentaire riche en graisses.

5. Bactérie destinée à être utilisée selon l'une quelconque des revendications précédentes, dans laquelle les bifidobactéries doivent être administrées en tant que composant d'un produit alimentaire, d'un supplément alimentaire ou d'une composition pharmaceutique.

6. Bactérie pour utilisation selon la revendication 5, dans laquelle les bifidobactéries doivent être administrées en tant que composant d'un yaourt.

7. Bactérie pour utilisation selon la revendication 5, dans laquelle les bifidobactéries doivent être administrées en tant que composant d'un supplément alimentaire.

8. Bactérie pour utilisation selon la revendication 5, dans laquelle les bifidobactéries doivent être administrées en tant que composant d'une composition pharmaceutique.

9. Bactérie pour utilisation selon l'une quelconque des revendications précédentes, dans laquelle les bifidobactéries doivent être administrées à une dose de 10⁹ à 10¹¹ UFC de bactéries/dose.

10. Bactérie pour utilisation selon la revendication 6, dans laquelle le yaourt contient de 10⁸ à 10¹² UFC des bactéries par dose.
